# EUROPEAN PATENT APPLICATION

(11) **EP 3 378 852 A1**
(43) Date of publication of application: **26.09.2018**
(21) Application number: 16865774.0
(22) Date of filing: 17.11.2016
(51) Int. Cl.: C07D 209/48, A61K 31/4035, A61K 9/14, A61K 9/20, A61K 9/26

(54) **AMORPHOUS FORM OF APREMILAST, PREPARATION METHOD THEREFOR, AND APPLICATION THEREOF**

(30) Priority: 19.11.2015 CN 201510798142; 28.12.2015 CN 201510997064
(71) Applicant: Changzhou Ainuoxinrui Pharmaceuticals, Ltd., Changzhou, Jiangsu 213022 (CN)
(72) Inventor: ZHANG, Xini, Green Brook, New Jersey 08812 (US); XIONG, Zhigang, Changzhou Jiangsu 213022 (CN); ZI, Chunpeng, Changzhou Jiangsu 213022 (CN); ZHANG, Sanfeng, Changzhou Jiangsu 213022 (CN); YANG, Qingkun, Changzhou Jiangsu 213022 (CN); ZHOU, Tao, Changzhou Jiangsu 213022 (CN)
(74) Representative: ABG Patentes, S.L.
(86) International application number: PCT/CN2016/106201
(87) International publication number: WO 2017/084597

(57) **Abstract**

The present invention relates to an amorphous form of Apemilast, its methods of preparation and its application, and the X-ray powder diffraction (XRPD) pattern of the amorphous form contains three broad peaks at a diffraction angle between 2.0 to 50.0 expressed in degrees 2θ, but not contain sharp diffraction peaks. The present invention also related to solid dispersion of amorphous Apremilast and its method of preparation, and it contains amorphous Apremilast and two or more pharmaceutical excipients, and the weight ratio of Apremlaist to all of pharmaceutical excipients is 1:0.1∼100. The amorphous form and amorphous solid dispersion of Apremilast of the present invention increases the solubility of Apremilast and improves bioavailability of the drug product. As compared to the existing crystalline forms of Apremilast, its solubility increases significantly, which improves body's absorption of the drug and makes it more efficacious in the clinical therapeutic treatment of diseases. Under the stress test conditions, the amorphous material can maintain good physical and chemical stabilities. The preparation method of amorphous Apremilast according to the present invention is simple to operate and easy to implement.

## Description

### Field of the Technology

The present invention is related to the field of drug formulation, in particular to an amorphous form of Apremilast and the method for its preparation, and a solid dispersion of amorphous Apremilast and the method for its preparation. The present invention also provides a pharmaceutical composition and the use of the above composition to prepare a medicament for the treatment of psoriatic arthritis and moderate to severe plaque psoriasis.

### Backgroud of the Invention

Apremilast has its chemical name as N-[2-[(1S)-1-(3-ethoxy-4-Methoxyphenyl)-2-(Methylsulfonyl)ethyl]-2,3-dihydro-1,3- dioxo-1H-isoindol-4-yl]- Acetamide, and its Brand name is OTEZLA. Its chemical structure is shown as Formula (I):

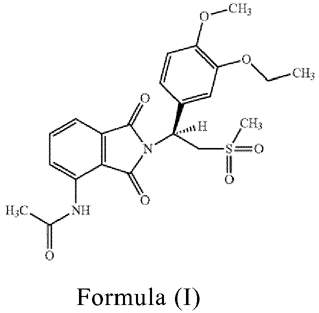

Apremilast is a small molecular phosphodiesterase 4 (PDE 4) inhibitor developed by Celgene Corporation, and was approved by the US Food and Drug Administration (FDA) for approval on March 21, 2014 for the treatment of adult patient's active psoriatic arthritis. The drug is the first and only one PDE4 inhibitor for the treatment of plaque psoriasis approved by FDA. The drug has good market response after marketing. The drug is also applying for a new indications for treatment of moderate to severe plaque psoriasis.

Apremilast has many crystal forms. The crystal form in market of Apremilast is anhydrous From B. US patent No. 7893101 disclosed the crystal forms of Apremilast: From A, From B, Form C, Form D, Form E, Form F and Form G. US patent No. 2015283249 disclosed an amorphous Apremilast, and provided XRPD pattern and the method for preparation.

The solid form of the drug directly affects the dissolution rate of the drug substance, the dissolution rate and the bioavailability of the drug substance. In order to improve the bioavailability of the drug, and hence reduce the dosage and reduce the side effects, new solid forms of the drug are usually developed. Therefore, to develop a solid form with higher drug solubility and better bioavailability is necessary.

The solid form of drug has not only crystalline form but also amorphous form. The amorphous form of a drug, as a special form of the solid material, plays an important role in the preparation of drugs. Because of the orderly periodic arrangement of molecules of crystalline substances, the energy of interaction of molecules reduces, and the energy is lower. However, the molecules of amorphous form is in a highly disordered state, so the free energy of surface is higher, and the molecules in amorphous form have higher energy than those in crystalline form. It is easier to disperse, increases dissolution and improve the bioavailability of drugs. Amorphous drugs can not only be widely used in pharmaceutical preparations, but also by a variety of technical approaches to improve the stability of amorphous drugs, making them the drugs with good quality.

US patent No. 2015283249 disclosed an amorphous form of Apremilast, but it was prepared by milling or spray drying. The milling needs a ball mill, and the spray drying needs a spray dryer. The above two methods have higher requirements for production equipment, so the production cost is high.

Due to the lack of bioavailability of Apremilast and the potential applications of amorphous active pharmaceutical ingredients in pharmaceutical preparations, it is very necessary to look for new amorphous Apremilast and its preparation method.

### Description of the Invention

The purpose of the present invention is to provide an amorphous form of Apremilast, method for preparation and its application thereof. It increases the solubility of Apremilast and improves availability of formulation.

In order to achieve the above goal, the technical solution of the present invention is as follows:
An amorphous form of Apremilast, using a Cu-Kα radiation, the amorphous Apremilast is characterized by the X-ray powder diffraction pattern without sharp diffraction peaks at a diffraction angle expressed in degrees 2θ.

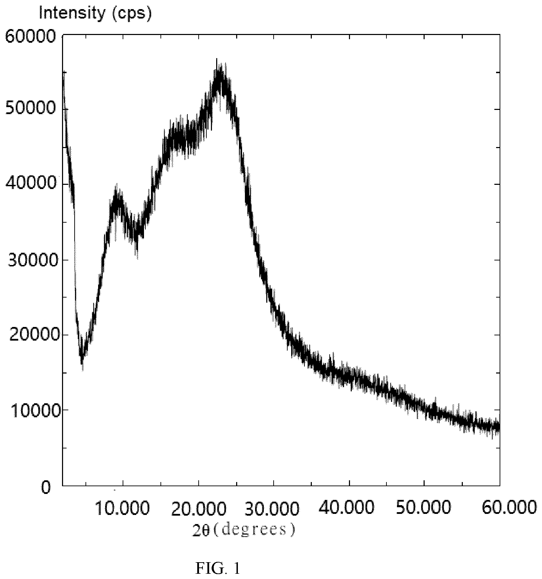

Preferably, the X-ray powder diffraction pattern has three broad peaks at a diffraction angle expressed in degrees between 2.0 and 50.0 degrees 2θ, and the X-ray powder diffraction is shown in Figure 1.

The present invention provides a method for preparing the amorphous form of Apremilast, comprising the steps of
1) dissolving Apremilast in solvent I to form a solution with a concentration of 0.01∼1 g/mL
2) adding the solution obtained from step 1) to solvent II at temperature - 80∼100°C to form a suspension, wherein solvent I is different from solvent II, and the volume ratio of the solution obtained from step 1) and solvent II is 1:1∼200
3) filtering the suspension formed in step 2) and drying the filter cake to obtain the amorphous form of Apremilast.

Preferably, solvent I is selected from at least one of alcohol, ketone, ether, halo hydrocarbon, amide, sulfone or sulfoxide, each containing 8 or fewer carbons; and solvent II is selected from at least one of hydrocarbon, aromatic hydrocarbon or water, each containing 8 or fewer carbons.

The present invention provides another method for preparing the amorphous form of Apremilast, comprising the steps of
1) dissolving Apremilast in solvent I to form a solution with a concentration of 0.01∼1 g/mL
2) removing solvent of the solution obtained from step 1) to obtain the amorphous form of Apremilast.

Future, the organic solvent is selected from at least one of alcohol, ether, ketone or nitrile, each containing 8 or fewer carbons.

Preferably, the method for removing solvent is the solvent evaporation method.

The amorphous form of Apremilast of the present invention is a new solid form. Because of the orderly periodic arrangement of molecules of crystalline substances, the energy of interaction of molecules reduces, and the energy is lower. However, the molecules of amorphous form is in a highly disordered state, and the molecules in amorphous form have higher energy than those in crystalline form. It is easier to disperse, and increases dissolution.

The amorphous form of the drug substance in the present invention has high dispersity. After forming the solid formulation, the dispersion of drug molecules is better and faster, which improves body's absorption of the drug. At the same time, compared to crystalline form, amorphous form is in a highly disordered state, and has higher surface free energy. Therefore, the solubility of amorphous form increases significantly, which improves body's absorption of the drug and makes it more efficient in the clinical treatment.

The present invention provides a solid dispersion of Apremilast and pharmaceutical excipient and the methods for preparation. The obtained solid dispersion of Apremilast and pharmaceutical excipient has good solubility and dispersity, which increases the dissolution rate. The method for preparation is not limited by the drying process, nor is it limited by the types of solvents or the amount of solvents. The method is easy to operate, low cost, easy to implement, and can achieve industrial production.

The present invention provides a solid dispersion of Apremilast and pharmaceutical excipient. The solid dispersion comprises Apremilast and two or more pharmaceutical excipients, and the weight ratio of Apremlaist to all of pharmaceutical excipients is 1:0.1∼100, wherein the Apremilast thereof in the solid dispersion is in an amorphous form, wherein in X-ray powder diffraction pattern of the composition, no characteristic peaks of crystalline Apremilast thereof are present after deducting background peaks of the pharmaceutical excipients.

Further, the pharmaceutical excipient is selected from at least one of diluent, lubricant, adhesive, disintegrant, surfactant, film-forming materials, coating materials or capsule materials.

Preferably, at least one of the pharmaceutical excipients being selected from hydroxypropylmethylcellulose, hydroxypropylcellulose, povidone, polyethylene glycol, ethylcellulose, liposomes, methacrylic acid copolymers, polyvinyl acetate, carboxymethylethylcellulose, carboxymethylcellulose phthalate, hydroxyethylcellulose methyl cellulose phthalate, hydroxypropylmethyl cellulose acetate succinate, polyacrylic resin, carbopol, alginate, carrageenan, carboxypolactone, gums, polyvinyl alcohol, pregelatinized starch, cross-linked starch, sodium carboxymethyl starch, dextrin, carboxypolymethylene, chitosan, collagen, cyclodextrin, lactose, galactose, D-mannitol, Sorbitol, xylitol, urea, citric acid, tartaric acid, fumaric acid, maleic acid or succinic acid.

The present invention provides a method for preparing the solid dispersion of Apremilast and pharmaceutical excipient, comprising the steps of
1) Mixing Apremilast and pharmaceutical excipient, and heating to melt pharmaceutical excipient; thereof, the weight ratio of Apremlaist to all of pharmaceutical excipients is 1:0.1∼100;
2) Cooling the mixture after mixing until uniform, and crushing the obtained mixture to obtain solid dispersion of amorphous Apremilast and pharmaceutical excipient.

Further, the pharmaceutical excipient is selected from at least one of diluent, lubricant, adhesive, disintegrant, surfactant, film-forming materials, coating materials or capsule materials.

Preferably, at least one of the pharmaceutical excipients being selected from hydroxypropylmethylcellulose, hydroxypropylcellulose, povidone, polyethylene glycol, ethylcellulose, liposomes, methacrylic acid copolymers, polyvinyl acetate, carboxymethylethylcellulose, carboxymethylcellulose phthalate, hydroxyethylcellulose methyl cellulose phthalate, hydroxypropylmethyl cellulose acetate succinate, polyacrylic resin, carbopol, alginate, carrageenan, carboxypolactone, gums, polyvinyl alcohol, pregelatinized starch, cross-linked starch, sodium carboxymethyl starch, dextrin, carboxypolymethylene, chitosan, collagen, cyclodextrin, lactose, galactose, D-mannitol, Sorbitol, xylitol, urea, citric acid, tartaric acid, fumaric acid, maleic acid or succinic acid.

The present invention provides another method for preparing the solid dispersion of Apremilast and pharmaceutical excipient, characterized by, comprising the steps of
1) mixing Apremilast and pharmaceutical excipient in solvent at the temperature between -50 and 150 °C, then forming the solution or suspension of Apremilast and pharmaceutical excipient, thereof, the weight ratio of Apremilast and solvent is 0.001∼100:1, and the weight ratio of Apremilast and all of excipients is 1:0.1∼100;
2) removing solvent of the solution or suspension obtained from step 1) to obtain the solid dispersion of Apremilast and pharmaceutical excipient.

Further, the pharmaceutical excipient is selected from at least one of diluent, lubricant, adhesive, disintegrant, surfactant, film-forming materials, coating materials or capsule materials.

Preferably, at least one of the pharmaceutical excipients being selected from hydroxypropylmethylcellulose, hydroxypropylcellulose, povidone, polyethylene glycol, ethylcellulose, liposomes, methacrylic acid copolymers, polyvinyl acetate, carboxymethylethylcellulose, carboxymethylcellulose phthalate, hydroxyethylcellulose methyl cellulose phthalate, hydroxypropylmethyl cellulose acetate succinate, polyacrylic resin, carbopol, alginate, carrageenan, carboxypolactone, gums, polyvinyl alcohol, pregelatinized starch, cross-linked starch, sodium carboxymethyl starch, dextrin, carboxypolymethylene, chitosan, collagen, cyclodextrin, lactose, galactose, D-mannitol, Sorbitol, xylitol, urea, citric acid, tartaric acid, fumaric acid, maleic acid or succinic acid.

Also, the solvent in step 1) is selected from at least one of alcohol, phenol, ether, halo hydrocarbon, ketone, aldehyde, nitrile, amide, sulfone, sulfoxide, carboxylic acid or water, each containing 12 or fewer carbons; the method for removing solvent in step 2) comprises evaporation, vacuum evaporation, spray drying, lyophilization, hot-melt extrusion, filtration, centrifugation or stirring film drying.

The present invention provides a pharmaceutical composition, and the pharmaceutical composition comprises amorphous Apremilast and at least two pharmaceutical excipients. The pharmaceutical excipient is selected from at least one of diluent, lubricant, adhesive, disintegrant, surfactant, film-forming materials, coating materials or capsule materials.

Further, at least one of the pharmaceutical excipients being selected from hydroxypropylmethylcellulose, hydroxypropylcellulose, povidone, polyethylene glycol, ethylcellulose, liposomes, methacrylic acid copolymers, polyvinyl acetate, carboxymethylethylcellulose, carboxymethylcellulose phthalate, hydroxyethylcellulose methyl cellulose phthalate, hydroxypropylmethyl cellulose acetate succinate, polyacrylic resin, carbopol, alginate, carrageenan, carboxypolactone, gums, polyvinyl alcohol, pregelatinized starch, cross-linked starch, sodium carboxymethyl starch, dextrin, carboxypolymethylene, chitosan, collagen, cyclodextrin, lactose, galactose, D-mannitol, Sorbitol, xylitol, urea, citric acid, tartaric acid, fumaric acid, maleic acid or succinic acid.

Also, any of the above compositions is used for the preparation of drugs for the treatment of psoriasis arthritis.

In addition, any of the above composition is used to prepare drugs for the treatment of moderate to severe plaque psoriasis.

Compared with the prior art, the beneficial effects of the present invention are as follows:
1) The present the amorphous form of Apremilast increases the solubility significantly, and the apparent solubility increases by more than 60% in most pH conditions, which improves bioavailability of drug and makes it more efficient in the clinical treatment. Under the accelerated conditions (40±2°C, humidity 75%±5%), the amorphous form can maintain good physical stability and chemical stability. Thus, the present invention will have broad applications. The method for preparation of amorphous Apremilast is simple to operate, easy to realize and can achieve industrial production.
2) The solid dispersion of amorphous form of Apremilast and two or more pharmaceutical excipients in the present invention has high dispersity and good stability. Various pharmaceutical excipients can play different roles in preparations of formulation, which is conducive to the development of pharmaceutical formulations. After forming the solid formulation, the dispersion of drug molecules is better and faster, which improves body's absorption of the drug. At the same time, compared to crystalline form, amorphous form is in a highly disordered state, and has higher surface free energy. Therefore, the solubility of amorphous form increases significantly, which improves body's absorption of the drug and makes it more efficient in the clinical treatment.
3) The method for preparation of the solid dispersion of amorphous form of Apremilast and pharmaceutical excipient is not limited by the drying process, nor is it limited by the types of solvents or the amount of solvents. The method is easy to operate, low cost, easy to implement, and can achieve industrial production.
4) In the solid dispersion of the amorphous form of Apremilast and pharmaceutical excipients prepared by the method of the present invention, the related substances have no significant change, and no Apremilast is crystallized out under the conditions of high temperature and high humidity; the related substances have no significant change, and no Apremilast is crystallized out under accelerated conditions (40 ± 2 °C, 75% ± 5% humidity). The solid dispersion of the amorphous form of Apremilast and pharmaceutical excipients in the present invention can maintain good physical stability and chemical stability and will have broad application prospects.

### Description of the Drawings

FIG. 1 is an X-ray powder diffraction pattern of the amorphous of Apremilast of Example 1 in the present invention.
FIG. 2 is a Thermal Gravimetric Analysis and Differential Scanning Calorimetry (TGA/DSC) diagram of the amorphous of Apremilast of Example 1 in the present invention.
FIG. 3 is an X-ray powder diffraction pattern of a solid dispersion of amorphous Apremilast, hydroxy propyl cellulose SSL and povidone K30 of Example 12 in the present invention.

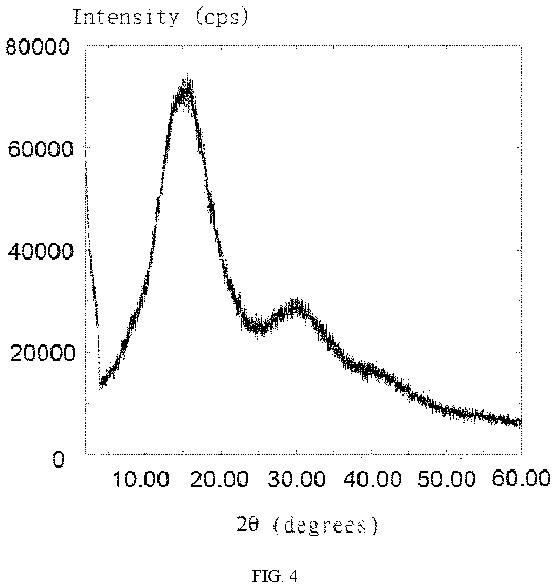

FIG. 4 is an X-ray powder diffraction pattern of a solid dispersion of amorphous Apremilast, sorbitol and polyacrylate Eudragit L100 of Example 23 in the present invention.

### Detailed Description

The present invention will be further described with reference to specific embodiments, but the protection scope of the present invention is not limited by the following embodiments.

The X-ray powder diffraction pattern according to the invention was taken on an Ultima IV X-ray diffractometer. The method parameters of the X-ray powder diffraction according to the present invention are as follows:
X-ray powder parameters: Cu-Kα
Kα (Å) : 1.5418;
Voltage: 40kV;
Current: 40mA;
Divergence slit: automatic;
Scanning mode: continuous;
Scanning range: from 2.0 to 60.0°;
Sampling period: 0.0200°;
Scanning rate: 60°/min.

The Thermal Gravimetric Analysis and Differential Scanning Calorimetry (TGA/DSC) diagram according to the invention was taken on a Q600 simultaneous Thermal Gravimetric Analyzer and Differential Scanning Calorimeter. The method parameters of the Differential Scanning Calorimetry according to the present invention are as follows:
Protective gas: Nitrogen
Temperature range: 20-800 °C
Scanning rate: 10 °C/min.

### Exsample 1:

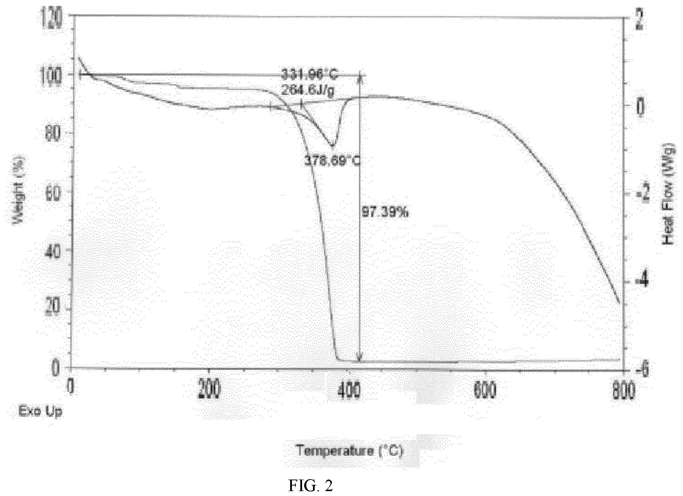

Apremilast (50 mg) was suspended in dichloromethane (750 µl), and the mixture was stirred to dissolve at the room temperature. The above solution was concentrated to dryness on a rotary evaporator with 5 minutes to obtain light yellow solid that was amorphous Apremilast. The X-ray Powder diffraction pattern was shown in FIG. 1, and three broad peaks at a diffraction angle between 2.0 to 50.0 expressed in degrees 2θ. The Thermal Gravimetric Analysis and Differential Scanning Calorimetry (TGA/DSC) diagram was shown in FIG. 2. It could be observed from FIG. 2: DSC comprises an endothermic event with an onset temperature of 339 °C, and TGA comprises loss of all mass after heating to 378 °C.

### Example 2:

Apremilast (50 mg) was suspended in tetrahydronfuran (750 µl), and the mixture was stirred to dissolve at the room temperature. The above solution was concentrated to dryness on a rotary evaporator with 5 minutes to obtain light yellow solid that was amorphous Apremilast.

### Example 3:

Apremilast (50 mg) was suspended in ethanol (5000 µl), and the mixture was stirred to dissolve at the room temperature. The above solution was concentrated to dryness on a rotary evaporator with 5 minutes to obtain light yellow solid that was amorphous Apremilast.

### Example 4:

Apremilast (50 mg) was suspended in acetonitrile (200 µl), and the mixture was stirred to dissolve at the room temperature. The above solution was concentrated to dryness on a rotary evaporator with 5 minutes to obtain light yellow solid that was amorphous Apremilast.

### Example 5:

Apremilast (50 mg) was added to acetonitrile (750 µl), and the mixture was stirred to dissolve at the room temperature. The above solution was added to n-heptane (10 ml) cooled to -80°C, and the yellow solid precipitated with stirring. After filtration and dryness, it was obtained solid (45 mg) that was amorphous Apremilast.

### Example 6:

Apremilast (30 mg) was added to N,N-dimethylformide (60 µl), and the mixture was stirred to dissolve at the room temperature. The above solution was added to n-heptane (10 ml) heated to 80°C, and the yellow solid precipitated with stirring. After filtration and dryness, it was obtained solid (27 mg) that was amorphous Apremilast.

### Example 7:

Apremilast (30 mg) was added to methanol (300 µl), and the mixture was stirred to dissolve at the room temperature. The above solution was added to water (1500 µl), and the yellow solid precipitated with stirring. After filtration and dryness, it was obtained solid (24 mg) that was amorphous Apremilast.

### Example 8:

Apremilast (30 mg) was added to acetone (300 µl), and the mixture was stirred to dissolve at the room temperature. The above solution was added to water (1500 µl), and the yellow solid precipitated with stirring. After filtration and dryness, it was obtained solid (25 mg) that was amorphous Apremilast.

### Example 9: Impact factor test of the amorphous Apremilast

### Material: Amorphous Apremilast was prepared according to the method of Example 1

### Experiment conditions: temperature 40 °C ± 2 °C, humidity 75% ± 5%

The substance was detected by HPLC.

The experiment results of impact factor test are shown in Table 1.

**Table 1**

| Detection items | Time | Experiment conditions | | | |
|---|---|---|---|---|---|
| | | Temperature 40 °C ± 2 °C | Temperature 60 °C ± 2 °C | Humidity 75%±5% | Humidity 90%±5% |
| Related substances (Total impurities %) | 0 day | 0.08 | 0.08 | 0.08 | 0.08 |
| | 5 days | 0.10 | 0.12 | 0.08 | 0.09 |
| | 10 days | 0.12 | 0.14 | 0.09 | 0.11 |
| crystalline (XRPD ) | 0 day | There is no peak of crystalline Apremilast | | | |
| | 5 days | There is no peak of crystalline Apremilast | | | |
| | 10 days | There is no peak of crystalline Apremilast | | | |

Table 1 shows that there is no significant changes in the related substances of amorphous Apremilast and no crystallization of Apremilast was observed in the conditions of high temperature, high humidity after 10 days.

### Example 10: Accelerated stability test of the amorphous Apremilast

Material: amorphous Apremilsat was prepared according to the method of Example 1.

### Experiment conditions: temperature 40 °C ± 2 °C, humidity 75% ± 5%

The substance was detected by HPLC.

The experiment results of accelerate stability test are shown in Table 2.

**Table 2**

| Detection items | Time | Experiment conditions |
|---|---|---|
| | | Temperature: 40°C±2°C, Humidity: 75%±5% |
| Related substances (Total impurities% ) | 0 month | 0.08 |
| | 1 month | 0.10 |
| | 2 months | 0.14 |
| | 3 months | 0.16 |
| | 6 months | 0.18 |
| Crystalline (XRPD) | 0 month | There is no peak of crystalline Apremilast |
| | 1 month | There is no peak of crystalline Apremilast |
| | 2 months | There is no peak of crystalline Apremilast |
| | 3 months | There is no peak of crystalline Apremilast |
| | 6 months | There is no peak of crystalline Apremilast |

Table 4 shows that there is no significant changes in the related substances of amorphous Apremilast and no crystallization of Apremilast was observed in the accelerated test conditions after 6 months.

The amorphous form of Apremilast in the present invention increases dissolution significantly, which improves body's absorption of the drug and makes it more efficient in the clinical treatment. The amorphous form can maintain good physical and chemical stability under the accelerated conditions (40±2°C, humidity 75%±5%).

### Example 11: Determination of Apparent solubility

The apparent solubility of the amorphous form of Apremilast in the present invention and the Apremilast crystal form is compared.

The test objects were as follows: the amorphous form of Apremilast obtained in Example 1 of the present invention; the crystal Form B of Apremilast was prepared according to the patent CN102702070 Example 12.

Determination of Apparent Solubility: the amorphous form of Apremilast obtained in Example 1 of the present invention and the crystal form B of Apremilast, respectively, was weighed into two plugged Erlenmeyer flasks, diluted with a predetermined pH value, Prepared as a supersaturated solution, tightly closed lid. Three samples were prepared in parallel in each pH dilution. Placed in 37 °C ± 0.5 °C shaking water bath shaker 12h, make it fully dissolved in order to achieve saturation. The supernatant with 0.45 micron microporous filter hot filter, and appropriate dilution, shake, were injected into the liquid chromatograph. The external standard method was used to calculate the apparent solubilities of three parallel samples in this pH buffer, averaged.

Preparation of diluents of different pH:
(1) pH=1.0 diluent: Add 9 mL of concentrated HCl to a 1000 mL of volumetric flask, diluted to the volume with water.
(2) pH=2.0 diluent: Solution A: Transfer 16.6 mL of phosphoric acid to a 100 mL volumetric flask, dilute to the volume with water, shake to mix. Solution B: Weigh and transfer 71.63g of sodium disodium phosphate to a 1000 mL of volumetric flask, dilute to the volume with water, dissolve and mix well. Mix 72.5 mL of solution A and 27.5 mL of solution B.
(3) pH=3.0 diluent: transfer 50 mL of glacial acetic acid to a 1000 mL of volumetric flask, add 800 mL of water, adjust pH to 3.0 with lithium hydroxide, then diluted to thevolume with water.
(4) pH=4.0 diluent: weigh and transfer 7.7 g of ammonium acetate, dissolve with 50 mL of water, add 6 mL of glacial acetic acid, then diluted to 100 mL with water.
(5) pH =5.6 diluent: Phthalate buffer (pH 5. 6): Transfer 10 g of potassium hydrogen phthalate, add 900 mL of water, stir to dissolve, adjust pH to 5.6 with sodium hydroxide solution (or diluted hydrochloric acid if necessary) then diluted to the volume with water.
(6) pH=6.8 diluent: mix 250 mL of 0.2 mol/L potassium dihydrogen phosphate solution and 118 mL of 0.2 mol/L sodium hydroxide solution , diluted to 1000 mL with water, shaken to mix.
(7) pH=7.4 diluent: transfer 1.36 g of potassium dihydrogen phosphate, add 79 mL of 0.1 mol/L sodium hydroxide solution, diluted to 200 mL with water.

The experimental results are shown in Table 3:

| pH value of Dilution | Apparent solubility (µg/ mL) | |
|---|---|---|
| | The amorphous form of Apremilast obtained in Example 1 | The crystal Form B of Apremilast |
| 2.0 | 19.32 | 7.86 |
| 3.0 | 66.78 | 48.12 |
| 4.5 | 42.23 | 24.18 |
| 5.0 | 22.81 | 9.99 |
| 5.6 | 30.02 | 16.71 |
| 6.8 | 23.19 | 12.53 |
| 7.4 | 18.71 | 10.71 |

Table 3 shows that at each pH, the apparent solubility of the amorphous form of Apremilast in the present invention is significantly higher than that of the crystal form.

The present the amorphous form of Apremilast increases the solubility significantly, which improves bioavailability of drug and makes it more efficient in the clinical treatment. The amorphous form can maintain good chemical and physical stabilities under accelerated conditions (temperature 40±2 °C, humidity 75%±5%).

### Example 12

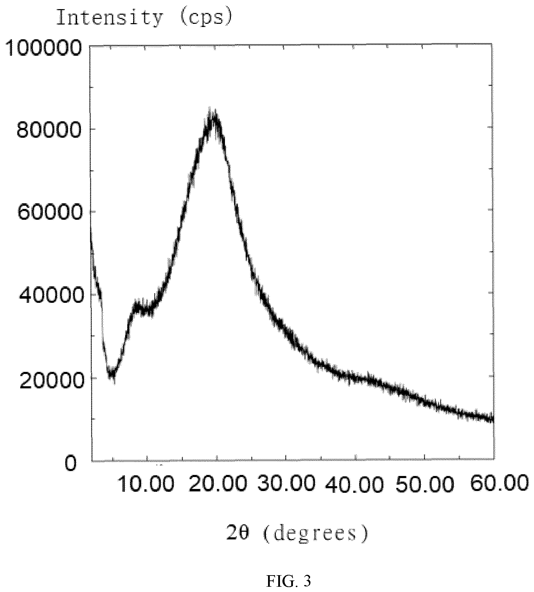

Apremilast (50 mg), hydroxypropyl cellulose SSL (50 mg) and povidone K30 (50 mg) were added to methanol (800 µl). The mixture was heated to 60 °C to dissolve with stirring. After removal of solvent in vacuum, it was obtained white solid that was the solid dispersion of amorphous Apremilast and hydroxypropyl cellulose SSL and povidone K30. The X-ray powder diffraction pattern of the solid dispersion was shown in FIG. 3, and the X-ray powder diffraction pattern of the solid dispersion does not contain characteristic peaks of Apremilast crystalline after deducting background peaks of pharmaceutical excipients.

### Example 13

Apremilast (50 mg), polyacrylic resin Eudragit L100 (50 mg) and polyethylene glycol 400 (200 mg) were added to ethanol (600 µl) and water (600 µl). The mixture stirred well at -40 °C. After removal of solvent in vacuum, it was obtained white solid that was the solid dispersion of amorphous Apremilast, Eudragit L100 and polyethylene glycol 400. The X-ray powder diffraction pattern of the solid dispersion does not contain characteristic peaks of Apremilast crystalline after deducting background peaks of pharmaceutical excipients.

### Example 14

Apremilast (2 g), lactose (2 g) and polyethylene glycol 8000 (10 g) were added to water (300 ml). The mixture was heated to 60 °C to dissolve with stirring. The above solution was spray dried with in JISL mini spray drier LSD-48 maintaining inlet temperature in range of 60 °C and inlet temperature in range of 50 °C. The product as white solid was collected, and it was further dried to obtain the solid dispersion of amorphous Apremilast, lactose and polyethylene glycol 8000. The X-ray powder diffraction pattern of the solid dispersion does not contain characteristic peaks of Apremilast crystalline after deducting background peaks of pharmaceutical excipients.

### Example 15

Apremilast (1 g), galactose (1 g) and hydroxypropyl methyl cellulose E50 (0.2 g) were added to water (10 ml). The mixture was heated to 40 °C to dissolve with stirring. The above solution was lyophilized to obtain white solid that was the solid dispersion of amorphous Apremilast, galactose and hydroxypropyl methyl cellulose E50. The X-ray powder diffraction pattern of the solid dispersion does not contain characteristic peaks of Apremilast crystalline after deducting background peaks of pharmaceutical excipients.

### Example 16

The mixture of Apremilast (5 g), urea (10 g) and polyethylene glycol 8000 (50 g) was heated to melt. Then it was cooled to room temperature rapidly to obtain white solid. The solid was crushed to obtain white powder that was the solid dispersion of amorphous Apremilast, urea and polyethylene glycol 8000. The X-ray powder diffraction pattern of the solid dispersion does not contain characteristic peaks of Apremilast crystalline after deducting background peaks of pharmaceutical excipients.

### Example 17

The mixture of Apremilast (1 g), ethanol (0.1 g), sorbitol (1 g) and polyethylene glycol 8000 (50 g) was heated to melt. Then it was cooled to room temperature rapidly to obtain white solid. The solid was crushed to obtain white powder that was the solid dispersion of amorphous Apremilast, urea, sorbitol and polyethylene glycol 8000. The X-ray powder diffraction pattern of the solid dispersion does not contain characteristic peaks of Apremilast crystalline after deducting background peaks of pharmaceutical excipients.

### Example 18

The mixture of Apremilast (1 g), fumaric acid (2 g), tetrahydrofuran (10 g), ethanol (20g) and liposome (4 g) were added to water (10 ml) was heated to 60 °C to mix well with stirring. After removal of solvent in vacuum, it was cooled to room temperature to obtain white solid that was the solid dispersion of amorphous Apremilast, fumaric acid and liposome. The X-ray powder diffraction pattern of the solid dispersion does not contain characteristic peaks of Apremilast crystalline after deducting background peaks of pharmaceutical excipients.

### Example 19

The mixture of Apremilast (1 g), methanol (20 g), polyacrylic resin Eudragit L100 (2 g) and methacrylic acid copolymer Type A was heated to 50 °C to dissolve with stirring. Then it was cooled to -30 °C rapidly to obtain white solid that was the solid dispersion of amorphous Apremilast, polyacrylic resin Eudragit L100 and methacrylic acid copolymer Type A. The X-ray powder diffraction pattern of the solid dispersion does not contain characteristic peaks of Apremilast crystalline after deducting background peaks of pharmaceutical excipients.

### Example 20

The mixture of Apremilast (1 g), methanol (20 g), pregelatinized starch (1 g) and ethylcellulose (2 g) was heated to 30 °C to mix well with stirring. After removal of solvent in vacuum, it was cooled to room temperature to obtain white solid that was the solid dispersion of amorphous Apremilast, pregelatinized starch and ethylcellulose. The X-ray powder diffraction pattern of the solid dispersion does not contain characteristic peaks of Apremilast crystalline after deducting background peaks of pharmaceutical excipients.

### Example 21

The mixture of Apremilast (1 g), methanol (20 g), xylitol (2 g) and hydroxypropyl cellulose SSL (4 g) was heated to 30 °C to mix well with stirring. After removal of solvent in vacuum, it was cooled to room temperature to obtain white solid that was the solid dispersion of amorphous Apremilast, xylitol and hydroxypropyl cellulose SSL. The X-ray powder diffraction pattern of the solid dispersion does not contain characteristic peaks of Apremilast crystalline after deducting background peaks of pharmaceutical excipients.

### Example 22

The mixture of Apremilast (1 g), methanol (20 g), water (10 g), citric acid (1 g) and polyvinyl acetate (4 g) was heated to 30 °C to mix well with stirring. After removal of solvent in vacuum, it was cooled to room temperature to obtain white solid that was the solid dispersion of amorphous Apremilast, citric acid and polyvinyl acetate. The X-ray powder diffraction pattern of the solid dispersion does not contain characteristic peaks of Apremilast crystalline after deducting background peaks of pharmaceutical excipients.

### Example 23

Apremilast (50 mg), sorbitol (100 mg) and polyacrylic resin Eudragit L100 (100 mg) was added to methanol (750µl), and the mixture dissolved with stirring at room temperature. After removal of solvent in vacuum, it was cooled to room temperature to obtain white solid that was the solid dispersion of amorphous Apremilast, sorbitol and polyacrylic resin Eudragit L100. The X-ray powder diffraction pattern of the solid dispersion does not contain characteristic peaks of Apremilast crystalline after deducting background peaks of pharmaceutical excipients.

### Example 24

Apremilast (50 mg), carboxymethylcellulose phthalate Agucoat CPD (20 mg) and polyacrylic resin Eudragit S100 (30 mg) was added to methanol (4 ml) and ethyl acetate (1 ml), and the mixture dissolved with stirring at -30 °C. After removal of solvent in vacuum, it was cooled to room temperature to obtain white solid that was the solid dispersion of amorphous Apremilast, carboxymethylcellulose phthalate Agucoat CPD and polyacrylic resin Eudragit S100. The X-ray powder diffraction pattern of the solid dispersion does not contain characteristic peaks of Apremilast crystalline after deducting background peaks of pharmaceutical excipients.

### Example 25

Apremilast (50 mg), dextrin (50 mg) and carboxypolymethylene Carbomer 940 (50 mg) was added to methanol (4 ml) and tetrahydrofuran (1 ml), and the mixture dissolved with stirring at -30 °C. After removal of solvent on a rotatory evaporator, it was cooled to room temperature to obtain white solid that was the solid dispersion of amorphous Apremilast, dextrin and carboxypolymethylene Carbomer 940. The X-ray powder diffraction pattern of the solid dispersion does not contain characteristic peaks of Apremilast crystalline after deducting background peaks of pharmaceutical excipients.

### Example 26

Apremilast (50 mg), β-dextrin (100 mg) and pregelatinized starch (100 mg) was added to methanol (4 ml) and water (1 ml), and the mixture dissolved with stirring at room temperature. After removal of solvent on a rotatory evaporator, it was cooled to room temperature to obtain white solid that was the solid dispersion of amorphous Apremilast, β-dextrin and pregelatinized starch. The X-ray powder diffraction pattern of the solid dispersion does not contain characteristic peaks of Apremilast crystalline after deducting background peaks of pharmaceutical excipients.

### Example 27

Apremilast (50 mg), β-dextrin (100 mg) and high amylopectin starch (50 mg) was added to methanol (4 ml) and water (1 ml), and the mixture dissolved with stirring at room temperature. After removal of solvent on a rotatory evaporator, it was cooled to room temperature to obtain white solid that was the solid dispersion of amorphous Apremilast, β-dextrin and high amylopectin starch. The X-ray powder diffraction pattern of the solid dispersion does not contain characteristic peaks of Apremilast crystalline after deducting background peaks of pharmaceutical excipients.

### Example 28

Apremilast (50 mg), maleic acid (100 mg) and sodium carboxymethylcellulose SCMC (500 mg) was added to dimethyl sulfoxide (5 ml), and the mixture dissolved with stirring at room temperature. After removal of solvent in vacuum, it was cooled to obtain white solid that was the solid dispersion of amorphous Apremilast, maleic acid and sodium carboxymethylcellulose SCMC. The X-ray powder diffraction pattern of the solid dispersion does not contain characteristic peaks of Apremilast crystalline after deducting background peaks of pharmaceutical excipients.

### Example 29

Apremilast (50 mg), polyethylene glycol 4000 (100 mg) and chitosan (400 mg) was added to ethanol (5 ml), and the mixture dissolved with stirring at room temperature. After removal of solvent on a rotatory evaporator, it was cooled to room temperature to obtain white solid that was the solid dispersion of amorphous Apremilast, polyethylene glycol 4000 and chitosan. The X-ray powder diffraction pattern of the solid dispersion does not contain characteristic peaks of Apremilast crystalline after deducting background peaks of pharmaceutical excipients.

### Example 30

Apremilast (50 mg), D-mannitol (100 mg) and sodium carboxymethyl starch Explotab (500 mg) was added to ethanol (5 ml), and the mixture dissolved with stirring at room temperature. After removal of solvent on a rotatory evaporator, it was cooled to room temperature to obtain white solid that was the solid dispersion of amorphous Apremilast, D-mannitol and sodium carboxymethyl starch Explotab. The X-ray powder diffraction pattern of the solid dispersion does not contain characteristic peaks of Apremilast crystalline after deducting background peaks of pharmaceutical excipients.

### Example 31

Apremilast (50 mg), povidone K90 (100 mg) and alginate E401 (50 mg) was added to ethanol (5 ml), and the mixture dissolved with stirring at room temperature. After removal of solvent on a rotatory evaporator, it was cooled to room temperature to obtain white solid that was the solid dispersion of amorphous Apremilast, povidone K90 and alginate E401. The X-ray powder diffraction pattern of the solid dispersion does not contain characteristic peaks of Apremilast crystalline after deducting background peaks of pharmaceutical excipients.

### Example 32

Apremilast (50 mg), L-tartaric acid (100 mg) and carboxymethylcellulose phthalate Agucoat CPD (1 g) suspended in methanol (30 ml), and the mixture was heated to 50 °C to dissolve with stirring. After removal of most of solvent on a rotatory evaporator, it was filtered and dried to obtain white solid that was the solid dispersion of amorphous Apremilast, L-tartaric acid and carboxymethylcellulose phthalate Agucoat CPD. The X-ray powder diffraction pattern of the solid dispersion does not contain characteristic peaks of Apremilast crystalline after deducting background peaks of pharmaceutical excipients.

### Example 33

Apremilast (50 mg), gum Galactosol (100 mg) and carrageenan E407 (100 mg) suspended in methanol (30 ml), and the mixture was heated to 50 °C to mix well with stirring. After removal of most of solvent on a rotatory evaporator, it was filtered and dried to obtain white solid that was the solid dispersion of amorphous Apremilast, gum Galactosol and carrageenan E407. The X-ray powder diffraction pattern of the solid dispersion does not contain characteristic peaks of Apremilast crystalline after deducting background peaks of pharmaceutical excipients.

### Example 34

Apremilast (50 mg), galactose (100 mg) and chitosan (200 mg) suspended in methanol (50 ml), and the mixture was heated to 50 °C to mix well with stirring. After removal of most of solvent on a rotatory evaporator, it was filtered and dried to obtain white solid that was the solid dispersion of amorphous Apremilast, galactose and chitosan. The X-ray powder diffraction pattern of the solid dispersion does not contain characteristic peaks of Apremilast crystalline after deducting background peaks of pharmaceutical excipients.

### Example 35

Apremilast (300 mg), liposome (300 mg) and polyacrylic resin Eudragit E100 (300 mg) dissolved in ethanol (600 µl), tetrahyfrofuran (900 µl) and N,N-dimethylformide (600 µl), and the mixture was heated to 50 °C to dissolve with stirring. The abve solution was cooled to -30 °C and the white solid precipitated. Then it was filtered and dried to obtain white solid that was the solid dispersion of amorphous Apremilast, liposome and polyacrylic resin Eudragit E100. The X-ray powder diffraction pattern of the solid dispersion does not contain characteristic peaks of Apremilast crystalline after deducting background peaks of pharmaceutical excipients.

### Example 36

Apremilast (30 mg), xylitol (30 mg) and collagen Peptan (200 mg) dissolved in ethanol (600 µl) and acetonitrile (600 µl), and the mixture was heated to 50 °C to dissolve with stirring. After removal of most of solvent on a rotatory evaporator, white solid precipitated. It was filtered and dried to obtain white solid that was the solid dispersion of amorphous Apremilast, xylitol and collagen Peptan. The X-ray powder diffraction pattern of the solid dispersion does not contain characteristic peaks of Apremilast crystalline after deducting background peaks of pharmaceutical excipients.

### Example 37

Apremilast (30 mg), D-mannitol (30 mg) and gum Galactosol (200 mg) dissolved in methanol (900 µl), and the mixture was heated to 50 °C to dissolve with stirring. After removal of solvent on a rotatory evaporator, it was obtain white solid that was the solid dispersion of amorphous Apremilast, D-mannitol and gum Galactosol. The X-ray powder diffraction pattern of the solid dispersion does not contain characteristic peaks of Apremilast crystalline after deducting background peaks of pharmaceutical excipients.

### Example 38

Apremilast (30 mg), chitosan (30 mg) and hydroxypropylmethylcellulose phthalate HPMCP (30 mg) dissolved in methanol (750 µl) and water (750 µl), and the mixture was heated to 80 °C to dissolve with stirring. After removal of solvent on a rotatory evaporator, it was obtain white solid that was the solid dispersion of amorphous Apremilast, chitosan and hydroxypropylmethylcellulose phthalate HPMCP. The X-ray powder diffraction pattern of the solid dispersion does not contain characteristic peaks of Apremilast crystalline after deducting background peaks of pharmaceutical excipients.

### Example 39

Apremilast (30 mg), D-mannitol (30 mg) and carboxypolactone (300 mg) were added to ethanol (750 µl) and water (750 µl), and the mixture was heated to 80 °C to mix well with stirring. After removal of solvent on a rotatory evaporator, it was obtain white solid that was the solid dispersion of amorphous Apremilast, D-mannitol and carboxypolactone. The X-ray powder diffraction pattern of the solid dispersion does not contain characteristic peaks of Apremilast crystalline after deducting background peaks of pharmaceutical excipients.

### Example 40

Apremilast (30 mg), β-dextrin (60 mg) and dextrin Maltrin M100 (60 mg) were added to ethanol (750 µl) and water (750 µl), and the mixture was heated to 80 °C to mix well with stirring. After removal of solvent on a rotatory evaporator, it was obtain brown solid that was the solid dispersion of amorphous Apremilast, β-dextrin and dextrin Maltrin M100. The X-ray powder diffraction pattern of the solid dispersion does not contain characteristic peaks of Apremilast crystalline after deducting background peaks of pharmaceutical excipients.

### Example 41

Apremilast (30 mg), succinic acid (3 mg) and sodium carboxymethyl cellulose (3 mg) were added to water (30 ml), and the mixture was heated to 100 °C to mix well with stirring. After removal of solvent on a rotatory evaporator, it was obtain white solid that was the solid dispersion of amorphous Apremilast, succinic acid and sodium carboxymethyl cellulose. The X-ray powder diffraction pattern of the solid dispersion does not contain characteristic peaks of Apremilast crystalline after deducting background peaks of pharmaceutical excipients.

### Example 42

Apremilast (5 mg), sorbitol (5 mg) and polyoxyethyene Polyox WSR301 (30 mg) were added to ethanol (300 µl) and water (60 µl), and the mixture was heated to 60 °C to mix well with stirring. After removal of solvent on a rotatory evaporator, it was obtain white solid that was the solid dispersion of amorphous Apremilast, sorbitol and polyoxyethyene Polyox WSR301. The X-ray powder diffraction pattern of the solid dispersion does not contain characteristic peaks of Apremilast crystalline after deducting background peaks of pharmaceutical excipients.

### Example 43

Apremilast (30 mg), sorbitol (20 mg), polyethylene glycol 8000 (20 mg) and polyvinyl alcohol EG-40 (200 mg) were added to methanol (300 µl) and water (60 µl), and the mixture was heated to 60 °C to mix well with stirring. After removal of solvent on a rotatory evaporator, it was obtain white solid that was the solid dispersion of amorphous Apremilast, sorbitol, polyethylene glycol 8000 and polyvinyl alcohol EG-40. The X-ray powder diffraction pattern of the solid dispersion does not contain characteristic peaks of Apremilast crystalline after deducting background peaks of pharmaceutical excipients.

### Example 44

Apremilast (50 mg), xylitol (50 mg) and hydroxypropylmethylcellulose acetate succinate Agoat MG (1 g) were added to ethanol 10 ml) and water (2 ml), and the mixture was heated to 80 °C to mix well with stirring. After removal of solvent on a rotatory evaporator, it was obtain white solid that was the solid dispersion of amorphous Apremilast, xylitol and hydroxypropylmethylcellulose acetate succinate Agoat MG. The X-ray powder diffraction pattern of the solid dispersion does not contain characteristic peaks of Apremilast crystalline after deducting background peaks of pharmaceutical excipients.

### Example 45

Apremilast (50 mg), sorbitol (100 mg) and carboxymethylethylcellulose (1 g) were added to ethanol 10 ml) and water (1 ml), and the mixture was heated to 80 °C to mix well with stirring. After removal of solvent on a rotatory evaporator, it was obtain white solid that was the solid dispersion of amorphous Apremilast, sorbitol and carboxymethylethylcellulose. The X-ray powder diffraction pattern of the solid dispersion does not contain characteristic peaks of Apremilast crystalline after deducting background peaks of pharmaceutical excipients.

### Example 46: Impact factor test of the solid dispersion of amorphous Apremilast and hydroxypropylcellulose SSL and povidone K30

### Material: the solid dispersion of amorphous Apremilast and hydroxypropylcellulose SSL and povidone K30 was prepared according to the method of Example 12

Experiment conditions: temperature 40 oC ± 2 oC, humidity 75% ± 5% The substance was detected by HPLC.

The experiment results of impact factor test are shown in Table 4.

**Table 4**

| Detection items | Time | Experiment conditions | | | |
|---|---|---|---|---|---|
| | | Temperature 40 °C ± 2 °C | Temperature 60 °C ± 2 °C | Humidity 75% ± 5% | Humidity 90% ± 5% |
| Related substances (Total impurities %) | 0 day | 0.08 | 0.08 | 0.08 | 0.08 |
| | 5 days | 0.10 | 0.11 | 0.08 | 0.09 |
| | 10 days | 0.12 | 0.14 | 0.09 | 0.11 |
| crystalline (XRPD) | 0 day | There is no peak of crystalline Apremilast | | | |
| | 5 days | There is no peak of crystalline Apremilast | | | |
| | 10 days | There is no peak of crystalline Apremilast | | | |

Table 4 shows that there is no significant changes in the related substances of the solid dispersion of amorphous Apremilast and hydroxypropylcellulose SSL and povidone K30 and no crystallization of Apremilsat was observed in the conditions of high temperature, high humidity after 10 days.

### Example 47: Accelerated stability test of the solid dispersion of amorphous Apremilast and hydroxypropylcellulose SSL and povidone K30

Material: the solid dispersion of amorphous Apremilast and hydroxypropylcellulose SSL and povidone K30 was prepared according to the method of Example 12.

### Experiment conditions: temperature 40 oC ± 2 oC, humidity 75% ± 5% The substance was detected by HPLC.

The experiment results of accelerate stability test are shown in Table 5.

**Table 5**

| Detection items | Time | Experiment conditions |
|---|---|---|
| | | Temperature: 40°C ± 2°C, Humidity: 75% ± 5% |
| Related substances (Total impurities% ) | 0 month | 0.08 |
| | 1 month | 0.11 |
| | 2 months | 0.14 |
| | 3 months | 0.15 |
| | 6 months | 0.18 |
| Crystalline (XRPD) | 0 month | There is no peak of crystalline Apremilast |
| | 1 month | There is no peak of crystalline Apremilast |
| | 2 months | There is no peak of crystalline Apremilast |
| | 3 months | There is no peak of crystalline Apremilast |
| | 6 months | There is no peak of crystalline Apremilast |

Table 4 shows that there is no significant changes in the related substances of the solid dispersion of amorphous Apremilast and hydroxypropylcellulose SSL and povidone K30 and no crystallization of Apremilast was observed in the accelerated test conditions after 6 months.

### Example 48: Determination of Apparent solubility

The apparent solubility of the solid dispersion of amorphous Apremilast in the present invention and the physical mixture comprising Apremilast crystal form is compared.

The test objects were as follows: the solid dispersion of amorphous Apremilast obtained in Example 12 of the present invention; the physical mixture comprising Apremilast crystalline (Apremilast crystal Form B, hydroxypropylcellulose SSL and povidone K30 were mixed physically, and the weight ratio is 1:1:1, and the crystal Form B of Apremilast was prepared according to the patent CN102702070 Example 12.).

Determination of Apparent Solubility: the amorphous form of Apremilast obtained in Example 1 of the present invention and the crystal form B of Apremilast, respectively, was weighed into two plugged Erlenmeyer flasks, diluted with a predetermined pH value, Prepared as a supersaturated solution, tightly closed lid. Three samples were prepared in parallel in each pH dilution. Placed in 37 °C ± 0.5 °C shaking water bath shaker 12 h, make it fully dissolved in order to achieve saturation. The supernatant with 0.45 micron microporous filter hot filter, and appropriate dilution, shake, were injected into the liquid chromatograph. The external standard method was used to calculate the apparent solubilities of three parallel samples in this pH buffer, averaged.

Preparation of diluents of different pH:
(1) pH=1.0 diluent: Add 9 mL of concentrated HCl to a 1000 mL of volumetric flask, diluted to the volume with water.
(2) pH=2.0 diluent: Solution A: Transfer 16.6 mL of phosphoric acid to a 100 mL volumetric flask, dilute to the volume with water, shake to mix. Solution B: Weigh and transfer 71.63g of sodium disodium phosphate to a 1000 mL of volumetric flask, dilute to the volume with water, dissolve and mix well. Mix 72.5 mL of solution A and 27.5 mL of solution B.
(3) pH=3.0 diluent: transfer 50 mL of glacial acetic acid to a 1000 mL of volumetric flask, add 800 mL of water, adjust pH to 3.0 with lithium hydroxide, then diluted to thevolume with water.
(4) pH=4.0 diluent: weigh and transfer 7.7 g of ammonium acetate, dissolve with 50 mL of water, add 6 mL of glacial acetic acid, then diluted to 100 mL with water.
(5) pH =5.6 diluent: Phthalate buffer (pH 5. 6): Transfer 10 g of potassium hydrogen phthalate, add 900 mL of water, stir to dissolve, adjust pH to 5.6 with sodium hydroxide solution (or diluted hydrochloric acid if necessary) then diluted to the volume with water.
(6) pH=6.8 diluent: mix 250 mL of 0.2 mol/L potassium dihydrogen phosphate solution and 118 mL of 0.2 mol/L sodium hydroxide solution , diluted to 1000 mL with water, shaken to mix.
(7) pH=7.4 diluent: transfer 1.36 g of potassium dihydrogen phosphate, add 79 mL of 0.1 mol/L sodium hydroxide solution, diluted to 200 mL with water.

The experimental results are shown in Table 6:

**Table 6**

| Dilution pH | Apparent solubility (µg / mL) | |
|---|---|---|
| | The solid dispersion obtained in Example 12 | The mixture comprising Apremilast crystalline |
| 2.0 | 17.57 | 7.64 |
| 3.0 | 66.13 | 44.46 |
| 4.5 | 38.25 | 26.81 |
| 5.0 | 20.81 | 9.91 |
| 5.6 | 28.99 | 15.56 |
| 6.8 | 22.39 | 11.58 |
| 7.4 | 16.02 | 10.02 |

Table 6 shows that at each pH, the apparent solubility of the solid dispersion of amorphous Apremilast obtained in Example 12 of the present invention is significantly higher than that of the physical mixture comprising Apremilast crystal Form B, hydroxypropylcellulose SSL and povidone K30.

### Example 49: Impact factor test of the solid dispersion of amorphous Apremilast, sorbitol and polyacrylic resin Eudragit L100

Material: the solid dispersion of amorphous Apremilast, sorbitol and polyacrylic resin Eudragit L100 was prepared according to the method of Example 23
Experiment conditions: temperature 40 °C ± 2 °C, humidity 75% ± 5% The substance was detected by HPLC.

The experiment results of impact factor test are shown in Table 7.

**Table 7**

| Detection items | Time | Experiment conditions | | | |
|---|---|---|---|---|---|
| | | Temperature 40 °C ± 2 °C | Temperature 60 °C ± 2 °C | Humidity 75% ± 5% | Humidity 90% ± 5% |
| Related substances (Total impurities %) | 0 day | 0.08 | 0.08 | 0.08 | 0.08 |
| | 5 days | 0.10 | 0.11 | 0.08 | 0.09 |
| | 10 days | 0.13 | 0.14 | 0.09 | 0.12 |
| crystalline (XRPD ) | 0 day | There is no peak of crystalline Apremilast | | | |
| | 5 days | There is no peak of crystalline Apremilast | | | |
| | 10 days | There is no peak of crystalline Apremilast | | | |

Table 7 shows that there is no significant changes in the related substances of the solid dispersion of amorphous Apremilast, sorbitol and polyacrylic resin Eudragit L100 and no crystallization of Apremilsat was observed in the conditions of high temperature, high humidity after 10 days.

### Example 50: Accelerated stability test of the solid dispersion of amorphous Apremilast sorbitol and polyacrylic resin Eudragit L100

Material: the solid dispersion of amorphous Apremilast sorbitol and polyacrylic resin Eudragit L100 was prepared according to the method of Example 23.

Experiment conditions: temperature 40 °C ± 2 °C, humidity 75% ± 5% The substance was detected by HPLC.

The experiment results of accelerate stability test are shown in Table 8.

**Table 8**

| Detection items | Time | Experiment conditions |
|---|---|---|
| | | Temperature: 40 °C ± 2 °C, |
| | | Humidity: 75%±5% |
| Related substances (Total impurities%) | 0 month | 0.08 |
| | 1 month | 0.11 |
| | 2 months | 0.13 |
| | 3 months | 0.15 |
| | 6 months | 0.17 |
| Crystalline (XRPD) | 0 month | There is no peak of crystalline Apremilast |
| | 1 month | There is no peak of crystalline Apremilast |
| | 2 months | There is no peak of crystalline Apremilast |
| | 3 months | There is no peak of crystalline Apremilast |
| | 6 months | There is no peak of crystalline Apremilast |

Table 4 shows that there is no significant changes in the related substances of the solid dispersion of amorphous Apremilast, sorbitol and polyacrylic resin Eudragit L100 and no crystallization of Apremilast was observed in the accelerated test conditions after 6 months.

### Example 51: Determination of Apparent solubility

The apparent solubility of the solid dispersion of amorphous Apremilast in the present invention and the physical mixture comprising Apremilast crystal form is compared.

The test objects were as follows: the solid dispersion of amorphous Apremilast obtained in Example 23 of the present invention; the physical mixture comprising Apremilast crystalline (Apremilast crystal Form B, sorbitol and polyacrylic resin Eudragit L100 were mixed physically, and the weight ratio is 1:1:1, and the crystal Form B of Apremilast was prepared according to the patent CN102702070 Example 12.).

Determination of Apparent Solubility: the amorphous form of Apremilast obtained in Example 1 of the present invention and the crystal form B of Apremilast, respectively, was weighed into two plugged Erlenmeyer flasks, diluted with a predetermined pH value, Prepared as a supersaturated solution, tightly closed lid. Three samples were prepared in parallel in each pH dilution. Placed in 37 °C ± 0.5 °C shaking water bath shaker 12 h, make it fully dissolved in order to achieve saturation. The supernatant with 0.45 micron microporous filter hot filter, and appropriate dilution, shake, were injected into the liquid chromatograph. The external standard method was used to calculate the apparent solubilities of three parallel samples in this pH buffer, averaged.

Preparation of diluents of different pH:
(1) pH=1.0 diluent: Add 9 mL of concentrated HCl to a 1000 mL of volumetric flask, diluted to the volume with water.
(2) pH=2.0 diluent: Solution A: Transfer 16.6 mL of phosphoric acid to a 100 mL volumetric flask, dilute to the volume with water, shake to mix. Solution B: Weigh and transfer 71.63g of sodium disodium phosphate to a 1000 mL of volumetric flask, dilute to the volume with water, dissolve and mix well. Mix 72.5 mL of solution A and 27.5 mL of solution B.
(3) pH=3.0 diluent: transfer 50 mL of glacial acetic acid to a 1000 mL of volumetric flask, add 800 mL of water, adjust pH to 3.0 with lithium hydroxide, then diluted to thevolume with water.
(4) pH=4.0 diluent: weigh and transfer 7.7 g of ammonium acetate, dissolve with 50 mL of water, add 6 mL of glacial acetic acid, then diluted to 100 mL with water.
(5) pH =5.6 diluent: Phthalate buffer (pH 5. 6): Transfer 10 g of potassium hydrogen phthalate, add 900 mL of water, stir to dissolve, adjust pH to 5.6 with sodium hydroxide solution (or diluted hydrochloric acid if necessary) then diluted to the volume with water.
(6) pH=6.8 diluent: mix 250 mL of 0.2 mol/L potassium dihydrogen phosphate solution and 118 mL of 0.2 mol/L sodium hydroxide solution , diluted to 1000 mL with water, shaken to mix.
(7) pH=7.4 diluent: transfer 1.36 g of potassium dihydrogen phosphate, add 79 mL of 0.1 mol/L sodium hydroxide solution, diluted to 200 mL with water.

The experimental results are shown in Table 6:

**Table 9**

| Dilution pH | Apparent solubility (µg / mL) | |
|---|---|---|
| | The solid dispersion obtained in Example 23 | The mixture comprising Apremilast crysalline |
| 2.0 | 17.57 | 7.64 |
| 3.0 | 66.13 | 44.46 |
| 4.5 | 38.25 | 26.81 |
| 5.0 | 20.81 | 9.91 |
| 5.6 | 28.99 | 15.56 |
| 6.8 | 22.39 | 11.58 |
| 7.4 | 16.02 | 10.02 |

Table 9 shows that at each pH, the apparent solubility of the solid dispersion of amorphous Apremilast obtained in Example 23 of the present invention is significantly higher than that of the physical mixture comprising Apremilast crystal Form B, sorbitol and polyacrylic resin Eudragit L100.

The solid dispersion of amorphous form of Apremilast and pharmaceutical excipients in the present invention increases dissolution significantly, which improves body's absorption of the drug and makes it more efficient in the clinical treatment. The amorphous form can maintain good physical and chemical stability under the accelerated conditions (40±2°C, humidity 75%±5%).

## Claims

1. An amorphous form of Apremilast, **characterized by**, using Cu-Kα radiation, an X-ray powder diffraction pattern expressed in degree 2θ not containing sharp diffraction peaks.

2. The amorphous form of Apremilast according to claim 1, **characterized by**, 5 the X-ray powder diffraction pattern comprising three broad peaks between 2.0 and 50.0 expressed in degrees 2θ.

3. A method for preparing the amorphous form of Apremilast according to claim 1 or 2, **characterized by**, comprising the steps of
1) dissolving Apremilast in solvent I to form a solution with a concentration of 0.01∼1g/mL
2) adding the solution obtained from step 1) to solvent II at temperature - 80∼100°C to form a suspension, wherein solvent I is different from solvent II, and the volume ratio of the solution obtained from step 1) and solvent II is 1:1∼200
3) filtering the suspension formed in step 2) and drying the filter cake to obtain the amorphous form of Apremilast.

4. The method for preparing the amorphous Apremilast according to claim 3, **characterized by**, solvent I being selected from at least one of alcohol, ketone, ether, halo hydrocarbon, amide, sulfone or sulfoxide, each containing 8 or fewer carbons.

5. The method for preparing the amorphous Apremilast according to claim 3, **characterized by**, solvent II being selected from at least one of hydrocarbon, aromatic hydrocarbon or water, each containing 8 or fewer carbons.

6. A method for preparing the amorphous form of Apremilast according to claim 1 or 2, **characterized by**, comprising the steps of
1) dissolving Apremilast in solvent I to form a solution with a concentration of 0.01∼1 g/mL
2) removing solvent of the solution obtained from step 1) to obtain the amorphous form of Apremilast.

7. The method for preparing the amorphous Apremilast according to claim 6, **characterized by**, the organic solvent being selected from at least one of alcohol, ether, ketone or nitrile, each containing 8 or fewer carbons.

8. The method for preparing the amorphous Apremilast according to claim 6, **characterized by**, the method for removing solvent in step 2) being solvent evaporation method.

9. A pharmaceutical composition of Apremilast, **characterized by**, the composition comprising amorphous Apremilast and at least one pharmaceutical excipients.

10. Use of the pharmaceutical composition of Apremilast according to claim 9 in the preparation of a medicament for the treatment of psoriatic arthritis and moderate to severe plaque psoriasis.

11. A solid dispersion of Apremilast and pharmaceutical excipient, **characterized by**, the solid dispersion comprising Apremilast and two or more pharmaceutical excipients, and the weight ratio of Apremlaist to all of pharmaceutical excipients is 1:0.1∼100, wherein the Apremilast thereof in the solid dispersion is in an amorphous form, wherein in X-ray powder diffraction pattern of the composition, no characteristic peaks of crystalline Apremilast thereof are present after deducting background peaks of the pharmaceutical excipients.

12. The solid dispersion of Apremilast and pharmaceutical excipient according to claim 11, **characterized by**, the pharmaceutical excipient being selected from at least one of diluent, lubricant, adhesive, disintegrant, surfactant, film-forming materials, coating materials or capsule materials.

13. The solid dispersion of Apremilast and pharmaceutical excipient according to claim 11, **characterized by**, at least one of the pharmaceutical excipients being selected from hydroxypropylmethylcellulose, hydroxypropylcellulose, povidone, polyethylene glycol, ethylcellulose, liposomes, methacrylic acid copolymers, polyvinyl acetate, carboxymethylethylcellulose, carboxymethylcellulose phthalate, hydroxyethylcellulose methyl cellulose phthalate, hydroxypropylmethyl cellulose acetate succinate, polyacrylic resin, carbopol, alginate, carrageenan, carboxypolactone, gums, polyvinyl alcohol, pregelatinized starch, cross-linked starch, sodium carboxymethyl starch, dextrin, carboxypolymethylene, chitosan, collagen, cyclodextrin, lactose, galactose, D-mannitol, Sorbitol, xylitol, urea, citric acid, tartaric acid, fumaric acid, maleic acid or succinic acid.

14. A method for preparing the solid dispersion of Apremilast and pharmaceutical excipient, **characterized by**, comprising the steps of
1) Mixing Apremilast and pharmaceutical excipient, and heating to melt pharmaceutical excipient; thereof, the weight ratio of Apremlaist to all of pharmaceutical excipients is 1:0.1∼100;
2) Cooling the mixture after mixing until uniform, and crushing the obtained mixture to obtain solid dispersion of amorphous Apremilast and pharmaceutical excipient.

15. The method for preparing the solid dispersion of Apremilast and pharmaceutical excipient according to claim 14, **characterized by**, the pharmaceutical excipient being selected from at least one of diluent, lubricant, adhesive, disintegrant, surfactant, film-forming materials, coating materials or capsule materials.

16. The method for preparing the solid dispersion of Apremilast and pharmaceutical excipient according to claim 14, **characterized by**, at least one of the pharmaceutical excipients being selected from hydroxypropylmethylcellulose, hydroxypropylcellulose, povidone, polyethylene glycol, ethylcellulose, liposomes, methacrylic acid copolymers, polyvinyl acetate, carboxymethylethylcellulose, carboxymethylcellulose phthalate, hydroxyethylcellulose methyl cellulose phthalate, hydroxypropylmethyl cellulose acetate succinate, polyacrylic resin, carbopol, alginate, carrageenan, carboxypolactone, gums, polyvinyl alcohol, pregelatinized starch, cross-linked starch, sodium carboxymethyl starch, dextrin, carboxypolymethylene, chitosan, collagen, cyclodextrin, lactose, galactose, D-mannitol, Sorbitol, xylitol, urea, citric acid, tartaric acid, fumaric acid, maleic acid or succinic acid.

17. A method for preparing the solid dispersion of Apremilast and pharmaceutical excipient, **characterized by**, comprising the steps of
1) mixing Apremilast and pharmaceutical excipient in solvent at the temperature between -50 and 150 °C, then forming the solution or suspension of Apremilast and pharmaceutical excipient, thereof, the weight ratio of Apremilast and solvent is 0.001∼100:1, and the weight ratio of Apremilast and all of excipients is 1:0.1∼100;
2) removing solvent of the solution or suspension obtained from step 1) to obtain the solid dispersion of Apremilast and pharmaceutical excipient.

18. The method for preparing the solid dispersion of Apremilast and pharmaceutical excipient according to claim 17, **characterized by**, the pharmaceutical excipient being selected from at least one of diluent, lubricant, adhesive, disintegrant, surfactant, film-forming materials, coating materials or capsule materials.

19. The method for preparing the solid dispersion of Apremilast and pharmaceutical excipient according to claim 17, **characterized by**, at least one of the pharmaceutical excipients being selected from hydroxypropylmethylcellulose, hydroxypropylcellulose, povidone, polyethylene glycol, ethylcellulose, liposomes, methacrylic acid copolymers, polyvinyl acetate, carboxymethylethylcellulose, carboxymethylcellulose phthalate, hydroxyethylcellulose methyl cellulose phthalate, hydroxypropylmethyl cellulose acetate succinate, polyacrylic resin, carbopol, alginate, carrageenan, carboxypolactone, gums, polyvinyl alcohol, pregelatinized starch, cross-linked starch, sodium carboxymethyl starch, dextrin, carboxypolymethylene, chitosan, collagen, cyclodextrin, lactose, galactose, D-mannitol, Sorbitol, xylitol, urea, citric acid, tartaric acid, fumaric acid, maleic acid or succinic acid.

20. The method for preparing the solid dispersion of Apremilast and pharmaceutical excipient according to claim 17, **characterized by**, the solvent in step 1) being selected from at least one of alcohol, phenol, ether, halo hydrocarbon, ketone, aldehyde, nitrile, amide, sulfone, sulfoxide, carboxylic acid or water, each containing 12 or fewer carbons; the method for removing solvent in step 2) comprising evaporation, vacuum evaporation, spray drying, lyophilization, hot-melt extrusion, filtration, centrifugation or stirring film drying.

21. A pharmaceutical composition of Apremilast, **characterized by**, the composition comprising amorphous Apremilast and two or more pharmaceutical excipients, and the pharmaceutical excipient being selected from at least one of diluent, lubricant, adhesive, disintegrant, surfactant, film-forming materials, coating materials or capsule materials.

22. The pharmaceutical composition of Apremilast, **characterized by**, at least one of the pharmaceutical excipients being selected from hydroxypropylmethylcellulose, hydroxypropylcellulose, povidone, polyethylene glycol, ethylcellulose, liposomes, methacrylic acid copolymers, polyvinyl acetate, carboxymethylethylcellulose, carboxymethylcellulose phthalate, hydroxyethylcellulose methyl cellulose phthalate, hydroxypropylmethyl cellulose acetate succinate, polyacrylic resin, carbopol, alginate, carrageenan, carboxypolactone, gums, polyvinyl alcohol, pregelatinized starch, cross-linked starch, sodium carboxymethyl starch, dextrin, carboxypolymethylene, chitosan, collagen, cyclodextrin, lactose, galactose, D-mannitol, Sorbitol, xylitol, urea, citric acid, tartaric acid, fumaric acid, maleic acid or succinic acid.

23. Use of the pharmaceutical composition of Apremilast according to claim 21 or 22 in the preparation of a medicament for the treatment of psoriatic arthritis.

24. Use of the pharmaceutical composition of Apremilast according to claim 21 or 22 in the preparation of a medicament for the treatment of moderate to severe plaque psoriasis.
